# EUROPEAN PATENT APPLICATION

(11) **EP 0 721 984 A1**
(43) Date of publication of application: **17.07.1996**
(21) Application number: 95200078.4
(22) Date of filing: 13.01.1995
(51) Int. Cl.: C12N 15/85, C12N 15/58, C12N 15/11

(54) **Enhancer element of gene expression**

(71) Applicant: Leuven Research & Development V.Z.W., B-3000 Leuven (BE); Collen, Désiré José, B-3020 Winkelsele-Herent (BE)
(72) Inventor: Collen, Désiré José Louis, B-3020 Winksele (BE); Belayew,Alexandra Pierre Janine Gaston, B-4130 Esneux (BE); Bulens, Frank Jan Maria Valeer, B-1950 Kraainem (BE)
(74) Representative: Van Someren, Petronella F. H. M.

(57) **Abstract**

The invention relates to an enhancer element suitable for inducing or enhancing the transcription of cis-located promoter/gene constructs in response to steroids an/or retinoids, comprising at least the region spanning nucleotides -8,007 to -7,144 upstream of the transcription initiation site of human tissue-type plasminogen activator, or modified versions thereof not destroying the biological activity of the element.

The invention also relates to a vector for the transfer of a gene or gene fragment into mammalian host cells, such that the gene or gene fragment may be expressed by the host cell, the vector comprising an enhancer element according to the invention operably linked to its homologous or any heterologous promoter an the gene or gene fragment.

## Description

The present invention relates to an enhancer element, e.g. useful for the inducible expression of therapeutic genes in vivo. The element may be incorporated in an appropriate gene therapy vector suitable for the induction or enhancement of transcription. The invention further relates to the use of such a vector and to a genetic switch comprising the enhancer element.

Recent advances in the understanding of the molecular basis of genetic disorders has led to the development of gene therapy for the treatment of inherited or acquired life threatening diseases. Gene therapy requires the introduction of the therapeutic gene of interest into somatic cells of the patient using a specific gene delivery system (e.g. liposomes, viruses; for a review see ref. 1). Expression of the therapeutic gene in the host cell will produce specific mRNA, which will be translated into active protein eventually leading to a specific and prolonged therapeutic effect in the treated patient.

Gene expression in general is primarily regulated at the level of transcription initiation by RNA polymerase II (2,3). Formation of the transcription initiation complex is favored by the presence of regulatory trans-acting factors that interact with specific cis-acting elements located in the promoter and in the enhancer(s) of the gene. The combination of various cis/trans modules, together with factors involved in protein-protein interactions, mediate a specific response and provide fine tuning to transcriptional regulation of a specific gene (2, 4-6).

In the case of gene therapy, usually strong viral promoter/enhancers are used for driving transcription of the therapeutic gene at a constitutively (high) level. Sometimes it might however be advantageous to be able to induce or increase transcription of the gene at will. This might for example be the case where a constitutive level of transcription is undesirable or transcription of the therapeutic gene depends on the presence of its homologous, but not very strong promotor.

It is therefore one of the objects of the present invention to provide an inducible enhancer element that might induce or increase transcription of a desired gene.

According to the invention it has now been found that the region spanning nucleotides -9,578 to -7,144 (2435 bp, called t-PA2.4) upstream of the transcription initiation site of the human tissue-type plasminogen activator (t-PA) gene enhances transcription of linked promoter/reporter gene constructs in response to (sub)pharmacological concentrations of steroid hormones and retinoic acid. The effect is observed in several cell lines, with several promoter/reporter constructs, it is synergistic and it is further enhanced by addition of a protein kinase C activator. The size of the enhancer could be reduced to a 862 bp fragment spanning nucleotides -8,007 to -7,145 upstream of the transcription initiation site of the human t-PA gene without significant loss of response to the various agents. The steroid responsiveness is located in the region spanning bp -7,325 to -7,535; it contains putative glucocorticoid responsive element(s) (GRE) that bind glucocorticoid and androgen receptor. The retinoic acid responsiveness is located in the region spanning nucleotides -7,145 to -7,324; it contains a retinoic acid responsive element (RARE) consisting of a GGGTCA direct repeat motif spaced by 5 nucleotides that interacts with heterodimers of retinoic acid receptor α and retinoid X receptor α.

The invention thus relates to an enhancer element suitable for inducing or enhancing the transcription of cis-located promotor/gene constructs in response to steroids and/or retinoids, comprising at least the region spanning nucleotides -8,007 to -7,145 upstream of the transcription initiation site of human tissue-type plasminogen activator, or modified versions thereof not destroying the biological activity of the element.

The enhancer element of the invention need not but may also comprise the region spanning nucleotides -9,578 to 7,144. Preferably the enhancer element comprises a steroid responsive fragment located in the region spanning nucleotides -7,325 to -7,535 and/or a retinoic acid responsive site located in the region spanning nucleotides - 7,145 to -7,324.

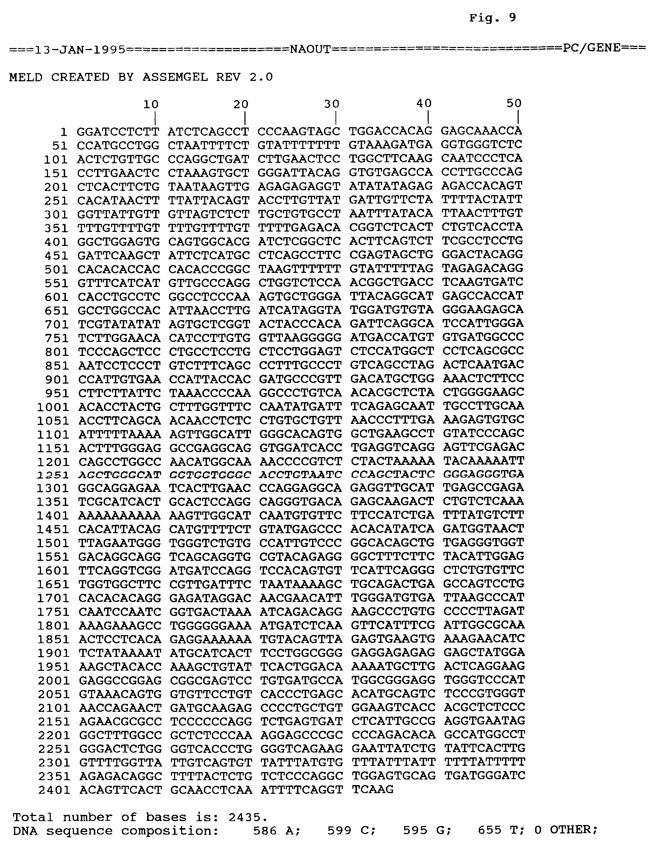

In one preferred embodiment of the invention the enhancer element comprises at least one biologically active part of the DNA sequence shown in Fig. 9, or modified versions thereof not destroying the biological activity of the element. The DNA fragment shown in Fig. 9 is further indicated with the term "t-PA2.4".

The synergistically inducible enhancer of the invention represents a valuable alternative to constitutive strong promoter/enhancers because by the nature of its synergistic response to various ligands, this enhancer could be linked to a weak promoter and provide a unique "genetic switch" for the induction or increase of the transcription of a therapeutic gene by (sub)pharmacological doses of steroid hormones, retinoic acid and activators of protein kinase C.

The invention thus provides for a genetic switch, comprising at least part of the enhancer element of the invention and a promoter having low or substantially no expression level of its own, wherein the enhancer element and the promoter are located on the same vector or on separate vectors to be used simultaneously. The term "genetic switch" is used to mean any construct of a promoter and the enhancer of the invention, wherein the promoter alone or without the enhancer being induced is so weak that the transcription level is low or substantially undetectable. Induction of the enhancer can then lead to a substantial increase of transcription level.

The t-PA2.4 enhancer or biologically active parts thereof, when incorporated in gene transfer vectors, will be useful for the in vivo expression of therapeutic genes in response to (sub)pharmacologic concentrations of steroid hormones, retinoic acid and activators of protein kinase C.

The invention thus further relates to a vector for the transfer of a gene or gene fragment into mammalian host cells, such that the gene or gene fragment may be expressed by the host cell, the vector comprising an enhancer element as claimed in any one of the claims 1 to 4, operably linked to its homologous or any heterologous promoter and the gene or gene fragment.

The vector of the invention may be used in the in vivo modulation of the expression of the gene or gene fragment in mammalian host cells, by natural or synthetic steroids, retinoids or combinations thereof, or by one or more agents acting on the enhancer element, such as activators of protein kinase C. The invention therefore also relates to these uses.

The present invention will be further elucidated by means of the following example, which is incorporated herein for illustration purposes only and is in no way intended to limit the scope of the invention.

### EXAMPLE

### Materials and methods

Human HT1080 fibrosarcoma, HeLa cervix carcinoma and SK-N-SH neuroblastoma cells were obtained from the American Type Culture collection (Rockville, MD, U.S.A.), and EA.hy926 endothelial hybrid cells from Dr. C.-J. Edgell (Dept. of Pathology, University of North Carolina, Chapel Hill, U.S.A.). 9cis-RA was a gift from Dr. F. Schneider (Hoffman-La Roche Ltd., Basel, Switzerland) expression vector encoding h-AR (RSV-hAR) from Dr. A.O. Brinkmann (Erasmus University, Rotterdam, the Netherlands), the λt-PAgen1 lambda genomic clone from Dr. L. Nelles (University of Leuven, Leuven, Belgium), expression vectors encoding h-ER (RSV-hER), h-PR (RSV-hPR), h-RAR_{α} (RAR_{α}φ), h-RAR_{β} (h-RAR_{β}) and h-RARγ (RARγ1.φ) from Dr. P. Chambon (Laboratoire de Génétique Moléculaire des Eukaryotes, Strasbourg, France), the expression vector encoding h-GR (RSV-hGR), h-MR (RSV-hMR) and h-RXR_{α} (pRSh-RXR_{α}) from Dr. R.M. Evans (The Salk Institute for biological Studies, La Jolla, CA, U.S.A.), the firefly luciferase expression vector (pRSVL) from Dr. D.R. Helinski (Department of Biology, University of California, San Diego, USA), purified fusion protein of protein A and the DNA binding domain of the human glucocorticoid and androgen receptor from Dr. P. De Vos (University of Leuven, Leuven, Belgium). epitope-tagged human RAR_{α} and RXR_{α} expression plasmids from Dr. T. Gulick and Dr. D.D. Moore (Massachusetts General Hospital, Boston, MA, USA) and epitope-tagged hRAR_{α} and hRXR_{α} as partially purified E. coli expression products from Dr. M. Baes (University of Leuven, Leuven, Belgium). Dulbecco's Modified Eagle growth medium (DMEM), DMEM-F12 and M199 growth medium and all medium supplements were purchased from Gibco/BRL (Ghent, Belgium), tissue culture recipients from corning Inc. (New York, USA) and Becton Dickenson (Franklin Lakes, NJ, USA), all-trans RA (RA), phorbol-ester-myristate (PMP), thymidine, dexamethasone (DEX), progesterone, d-aldosterone, 17β-estradiol, and chloramphenicol from Sigma Chem. Co. (St. Louis, MO, USA), the human genomic λEMBL3 library from Clontech (Palo Alto, CA, USA), DNA purification columns used for plasmid preparations from Qiagen (Chatsworth, CA, USA) and Mackerey-Nagel (Düren, Germany), all reagents used for DNA dideoxy sequencing reactions from Pharmacia (Roosendaal, The Netherlands), acetyl CoA and [³H]-acetyl CoA from ICN Biomedicals (Costa Mesa, CA, USA), [α³²P]UTP and [α³²P]dCTP from Amersham Corp. (Ghent, Belgium), SP6 RNA polymerase, pSP64 plasmid, the Erase-a-base kit, reporter lysis buffer and luciferin substrate from Promega (Madison, WI, USA), Galacton and "Galacto-Light" kit from Tropix (Bedford, Mass, USA), the synthetic androgen R1881 and Econofluor-2 from NEN-Dupont (Boston, MA, USA) and Lipoluma from Lumac-LSC (Olen, Belgium).

### DNA sequence analysis

For DNA dideoxy sequencing reactions the AutoRead Sequencing kit of Pharmacia Biotech was used with either fluorescent labeled or unlabeled primers in combination with fluorescent labeled dATP. Reactions were run on a 6% polyacrylamide-urea gel (0.6 x Tris borate buffer) using the Automated Laser Fluorescent DNA sequencer system from Pharmacia Biotech. Generated sequences were analyzed using the PC Gene software from Intelligenetics (Mountain View, CA, USA)

### Cell culture

Cells were grown in DMEM (HT1080, HeLa cells and EA.hy926 cells), DMEM-F12 (SK-N-SK cells) supplemented with glutamine (1 mM), penicillin (100 IU/ml), streptomycin (100 µg/ml) and 10% heat-inactivated fetal calf serum. M199 medium contained in addition 10% heat-inactivated human serum. Cells were seeded at a density of 2-4 x 10⁴ cells/cm² and grown overnight in DMEM with 5% heat-inactivated, charcoal-stripped fetal calf serum. RA and 9cis-RA were dissolved in dimethylsulfoxide or ethanol at a concentration of 10 to 100 mM and stored at -80°C. Phorbol-ester-myristate (PMA), dexamethasone, progesterone, d-aldosterone, the synthetic androgen R1881 and 17β-estradiol were dissolved in ethanol at a concentration of 10 to 100 mM. These agents were stored at -80°C and the appropriate concentration was added to the medium in a volume corresponding to 0.1% of the culture medium. Control medium contained an equal amount of excipient.

### Assays for t-PA, u-PA and PAI-1 antigens

t-PA, u-PA and PAI-1-related antigens in the conditioned medium were determined by specific enzyme-linked immunosorbent assays (7-9).

### Reporter constructs

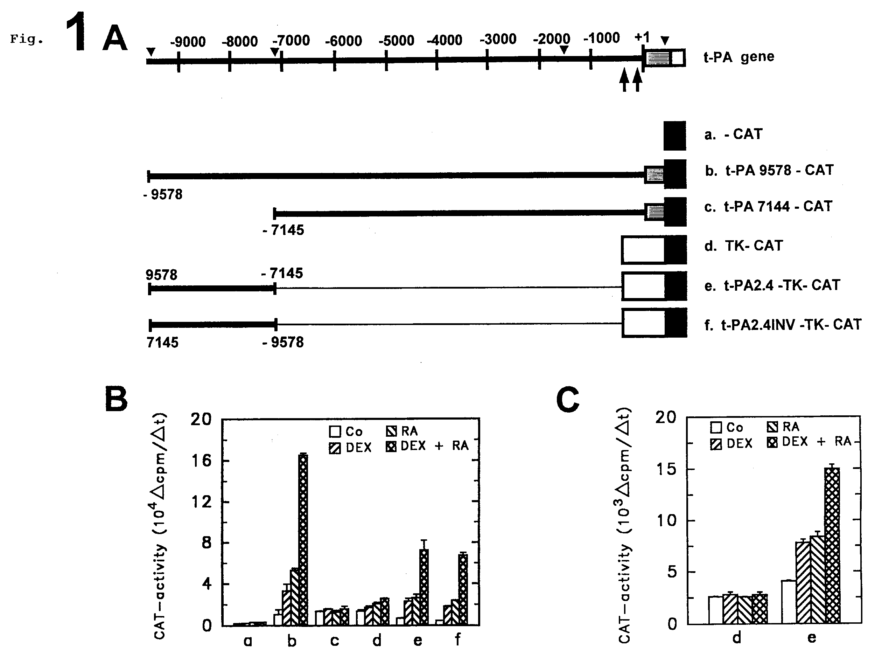

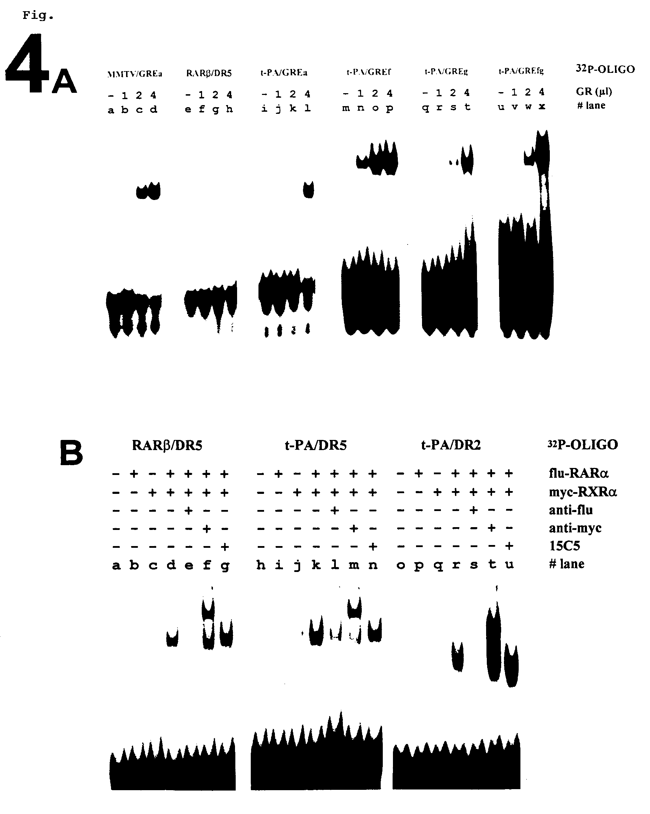

A recombinant lambda phage which contained 22 kb of human t-PA genomic sequence including 17 kb of upstream region (λt-PA gen1), was isolated from a human genomic λEMBL3 library using a human t-PA cDNA probe (10). All genomic sequences analyzed in this study are numbered relative to the transcription initiation site as determined by Henderson and Sleigh (11) and are schematically presented in figure 1A. A 7.3 kb (+197 to -7,144 bp) and 9.8 kb upstream fragment (+197 to -9,578 bp), obtained by partial BamHI digestion, were linked to the chloramphenicol acetyltransferase (CAT) gene in the reporter plasmid pBLCAT3 (12) to yield t-PA7144-CAT and t-PA9578-CAT, respectively. Deletion mutants t-PA632-CAT, t-PA1564-CAT and t-PA3070-CAT with respectively 632, 1564 and 3070 bp of t-PA gene upstream sequence were obtained from the t-PA7144-CAT construct by progressive exonuclease III deletion mutagenesis (Erase-a-base kit). The 2.4 kb BamHI upstream genomic fragment (-7,145 to -9,578 bp: t-PA2.4) was cloned in front of these constructs yielding t-PA632∇2.4-CAT, t-PA1564∇2.4-CAT and t-PA3070∇2.4-CAT, respectively (cfr. Fig. 5A) and in either orientation in front of the thymidine kinase(TK)-promoter linked to the CAT-gene in p BLCAT2 (12) yielding t-PA2.4-TK-CAT and t-PA2.4INV-TK-CAT (cfr. Figure 1A).

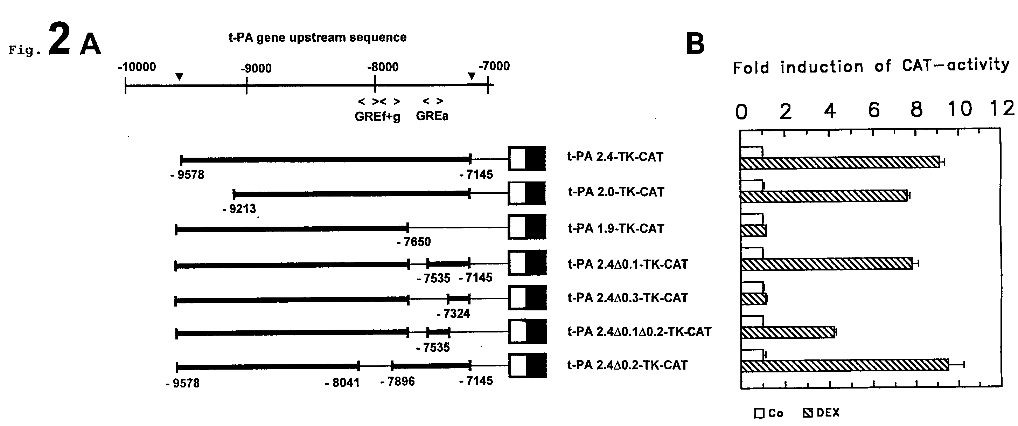

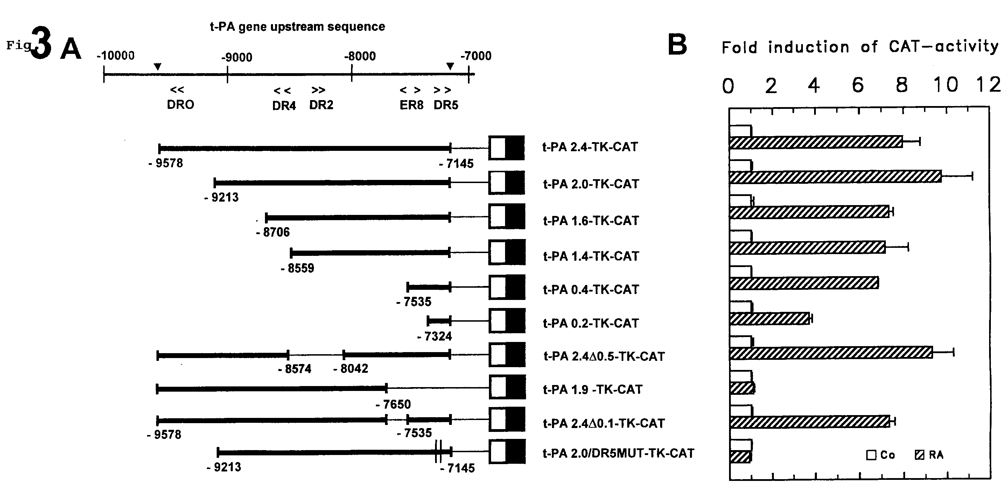

Constructs obtained by progressive exonuclease III deletion mutagenesis from the t-PA2.4-TK-CAT construct (t-PA2.0-TK-CAT, t-PA1.6-TK-CAT, t-PA1.4-TK-CAT, t-PA0.4-TK-CAT and t-PA0.2-TK-CAT) and from t-PA2.4INV-TK-CAT (t-PA1.9TK-CAT) are shown in figure 2A and 3A. Internal deletions were created by recombining deletion fragments from t-PA2.4-TK-CAT and from t-PA2.4INV-TK-cat, yielding the following constructs (Figure 2A, 3A): t-PA2.4△0.5-TK-CAT, t-PA2.4Δ0.1-TK-CAT, t-PA2.4Δ0.3-TK-CAT, t-PA2.4Δ0.1Δ0.2-TK-CAT, t-PA2.4Δ0.2-TK-CAT. The presumed DR5 RARE identified in the upstream 2.4 kb t-PA gene fragment was mutated (t-PA/DR5MUT; cfr. Table 1) in the t-PA2.0-TK-CAT construct by site-specific mutagenesis using PCR (13), yielding t-PA2.0/DR5MUT-TK-CAT.

DNA oligonucleotides representing recognition motifs for distinct nuclear receptors are shown in Table 1, some of which were cloned as two copies in front of the TK promoter linked to the CAT gene (t-PA/DR5-TK-CAT, t-PA/DR2-TK-CAT, RARβ/DR5-TK-CAT, MMTV/GREa-TK-CAT).

### Transfection analysis

To obtain stable expression of t-PA-CAT constructs in HT1080 cells, the calcium phosphate coprecipitation method (14) was applied using a DNA mixture which contained 20 to 60 µg of PvuI-linearized CAT reporter plasmid (quantity was according to the plasmid size) and 4 µg of PvuI-linearized pCMβNeo selection plasmid (15, 1/5 ratio). After 36-38 h of incubation, neomycin was added to the medium (500 µg/ml final concentration) and selection was performed for approximately 10 days. In order to avoid positional effects on the CAT reporter expression, experiments were performed using a large pool of neomycin resistant colonies (routinely 10²-10³). After growing to confluency, cells were harvested and seeded in 6-well dishes in DMEM medium containing 0.25% (w/v) bovine serum albumin and 0.5% of charcoal-stripped serum. After overnight incubation, the medium was replaced with medium containing DEX and/or RA or control solution. After 24 h of incubation, cells were analyzed as described below.

To obtain transient expression of the t-PA promoter constructs in HT1080, HeLa and SK-N-SH cells, the calcium phosphate coprecipitation method (14) was applied to a six well dish using a DNA mixture of 12 to 46 µg CAT reporter plasmid (according to the size of the reporter construct) with 0.1 µg pRSVL plasmid and 2.4 µg of the indicated nuclear receptor expression plasmid. Cells were stimulated with DEX and/or RA immediately (treatment for 36 hours) or 16 hours after the glycerol shock (treatment for 24 or 30 hours).

EA.hy926 cell were transiently transfected by electroporation according to a (modified) procedure described by Schwachtgen et al. (16). 10⁷ synchronized cells suspended in cytomix (17) were added to approximately 50 µg of PvuI-linearized reporter plasmid together with XmnI-linearized h-RARB and with NdeI-linearized h-RXRα expression vectors (ratio 20/1) followed by electroporation at a voltage of 274 V and a capacitance of 1,800 µF (time constant τ= 26 ± 0.3 ms, mean ± SEM, n= 10). Cells were immediately added to 12 ml of supplemented DMEM medium containing 5% of charcoal-stripped serum and divided in six 10 cm² wells. After overnight incubation, cells were washed with PBS and growth medium containing RA and/or PMA or excipient was added, followed by a 30 h incubation.

All cell extracts were prepared by three freeze-thaw cycles (Tris.HCl 100 mM pH 7.8, EDTA 5 mM) or by using reporter lysis buffer. CAT activity was quantified using the liquid scintillation method (18): a mixture of [³H]-acetyl CoA (0.1 µCi), acetyl CoA (final concentration 0.1 mM) and chloramphenicol (final concentration 0.9 mM) was added to equal amounts of cell extracts, overlayered with scintillation solution (either Econofluor-2 or Lipoluma) and the rate of [³H]-labeled acetyl-chloramphenicol generation was measured using a liquid scintillation analyzer. Luciferase or β-galactosidase-activity, used as an indicator for the transfection efficiency, was measured in a luminometer after addition of luciferin or "Galacton" chemiluminescent substrate, respectively. Data obtained from stable and transient expression experiments were corrected for endogenous CAT activity and luciferase activity.

### Electrophoretic mobility shift assay

DNA oligonucleotides or DNA fragments were labeled by filling in with the Klenow fragment of DNA polymerase I using [α³²P]dCTP. Electrophoretic mobility shift assays (EMSA) were performed according to Fried and Crothers (19) as modified by Baes et al. (20): RAR_{α} (NH₂-terminally tagged with the Hemophilus influenzae agglutinin nonapeptide: flu-RAR_{α}) and/or RXR_{α} (tagged with an epitope from c-MYC: myc-RXR_{α}) were incubated for 15 min at 25°C, added to the reaction mixture containing 10,000 cpm of labeled DNA fragment or oligonucleotide which was then incubated at 25°C for 30 min. Specific monoclonal antibodies directed against the flu-epitope (anti-flu) or the c-MYC epitope (anti-myc) were added to the reaction mixture as preformed complex with their respective epitope tagged receptor (flu-RAR_{α} or myc-RXR_{α}). As a negative control, the fibrin D-dimer directed 15C5 monoclonal antibody (21) was pre-incubated with either h-RAR_{α} or h-RXR_{α}. Band shift reactions were analyzed on a 4-5% polyacrylamide gel electrophoresis at 4°C in 0.5 x Tris borate buffer. Bands were visualized by autoradiography. EMSA with purified fusion protein of protein A- and the DNA-binding domain of the glucocorticoid (GR, 22) or the androgen receptor (AR, 23) was performed similarly as described above, except that GR or AR were directly added to the reaction mixture.

### Results

### Synergistic induction of t-PA gene expression in HT1080 cells by DEX and RA

The influence of a combined treatment of DEX with RA on t-PA, urinary-type plasminogen activator (u-PA) and plasminogen activator inhibitor (PAl)-1-related antigen secretion is summarized in Table 2. Whereas DEX induced an increase of t-PA as well as PAl-1-related antigen (2.9 ± 0.1 and 2.4 ± 0.1-fold, respectively) the secretion of u-PA-related antigen secretion was reduced by 0.64 ± 0.02-fold. RA alone induced a major increase of t-PA-related antigen secretion (11 ± 0.4-fold) but had only a 2.1 ± 0.7-fold effect on u-PA-related antigen secretion and no or very little effect on PAl-1 related antigen secretion. A combined treatment with DEX and RA had a cooperative effect on t-PA-related antigen secretion (35 ± 2.0-fold) as compared to the effect of these agents separately (cfr. above). The observation was specific for t-PA-related antigen as the u-PA-related antigen level remained unaffected (0.86 ± 0.05-fold) and only a small increase was seen for the PAl-1-related antigen level (3.0 ± 0.13-fold).

Northern blot analysis of total RNA extracted from HT1080 cells showed that the effects of these agents on the level of antigen secretion were also seen on their respective steady state mRNA levels (data not shown).

### DNA sequence determination of the human t-PA gene upstream sequences from bp +197 to -9,578

In order to study the cis-elements involved in the hormonal regulation of human t-PA gene expression, a recombinant lambda phage, containing 22 kb of human t-PA genomic sequence including 17 kb of upstream region, was isolated from a human λEMBL3 library in the Center for Molecular and Vascular Biology. A 9.8 kb upstream fragment (+197 to -9,578) was obtained by partial BamHl digestion and cloned in the pBLCAT3 reporter plasmid (12), yielding t-PA9578-CAT. Using a set of 5' progressive deletions derived from the t-PA9578-CAT construct in combination with 'primer walking' (data not shown) the DNA sequence upstream of the human t-PA gene from bp +197 to -9,578 relative to the start site of transcription (11) was determined in both directions as described in the Materials and Method section. The DNA sequence from nucleotide -7145 to -9578 relative to the start site of transcription (11) is added as Fig. 9. This sequence contained several palindromic (partial) repeats representing putative binding sites for the glucocorticoid receptor (5). In addition, 
A   G   G
  G   T   A
G   T   C
motifs which represent the sequence of the consensus recognition half-site for the RA-thyroid hormone nuclear receptor family were identified (6). Motifs resembling such consensus repeats are shown and described in Table 1.

### Localization of an enhancer responsive to DEX and RA 7 kb upstream from the human t-PA gene

t-PA promoter/CAT reporter vectors are schematically represented in Figure 1A, the CRE/AP1 and AP2 binding sites involved in basal promoter activity (26) are indicated (for details see Materials and Methods). Results obtained by stable integration of these reporter vectors in HT1080 cells, are shown in Figure 1B. t-PA9578-CAT reporter activity was 3.7 ± 0.1-fold and 6.2 ± 0.2-fold induced by DEX and RA, respectively, whereas the combination had a 21.0 ± 0.2-fold effect (cfr. Figure 1B). The t-PA7144-CAT construct showed no response to DEX and RA. t-PA2.4-TK-CAT was 5.1 ± 0.14-fold and 5.5 ± 0.5-fold induced by, respectively, DEX and RA alone, whereas the combination had a 18 ± 1.5-fold effect. Similar effects were observed for the t-PA2.4INV-TK-CAT construct, while the TK promoter alone (TK-CAT) showed little or no induction. Evaluation of the effect of (low) equipotent doses of DEX (10⁻⁸M) and RA (10⁻⁹M) revealed a 2.0 ± 0.1-fold induction of the t-PA2.4-TK-CAT construct compared to a significantly higher 3.7 ± 0.1-fold induction when 5x10⁻⁹ M DEX was combined with 5x10⁻¹⁰ M RA (cfr. Figure 1C).

In aggregate, these results show that a genomic fragment spanning the region from -7145 to -9578 bp upstream from the human t-PA gene can provide a synergistic transactivation in response to DEX and RA when linked to either its homologous or a heterologous promoter.

### Identification of the cis-acting elements involved in the response of the t-PA2.4 enhancer to DEX and RA

### 1. Identification of the DEX-responsive region at -7.5 kb

Figure 2A provides a schematic overview of the t-PA genomic sequence spanning -7,145 to -9,578 bp. The indicated putative glucocorticoid receptor binding sites (t-PA/GREa, t-PA/GREf and t-PA/GREg) are described in Table 1. Figure 2B represents the results of transient co-expression of t-PA2.4-TK-CAT mutants with the human glucocorticoid receptor (GR) in Hela cervix carcinoma cells. An internal deletion spanning the region from -8,042 to -7,897 bp (t-PA2.4Δ0.2-TK-CAT), which eliminated t-PA/GREf and t-PA/GREg (cfr. Table 1), had no effect on the induction of CAT-activity as compared to the t-PA2.4-TK-CAT wild-type construct (respectively 10 ± 1.6-fold and 9.1 ± 0.25-fold). Elimination of the t-PA/GREa element by deletion of either the region from -7,650 to -7,145 or from -7,650 to -7,324 bp (respectively t-PA1.9-TK-CAT and t-PA2.4Δ0.3-TK-CAT) abolished the induction. Re-introduction of most of the deleted region (-7,535 bp to 7,145 bp) or only the -7535 to -7,324 bp fragment, both of which contained the t-PA/GREa element, into the t-PA1.9-TK-CAT construct restored the induction (respectively, t-PA2.4Δ0.1-TK-CAT, 7.8 ± 0.29-fold and t-PA2.4Δ0.1Δ0.2-TK-CAT, 4.2 ± 0.09-fold).

In conclusion, these results confined the DEX responsive sequence to the region from -7,535 to -7,325 bp (t-PA0.2A) upstream of the human t-PA gene.

### 2. Identification of a DR5 RARE at -7.3 kb

Transient co-expression in HT1080 cells of the t-PA2.4-TK-CAT construct with expression plasmids encoding RA nuclear receptors increased CAT expression by RA and 9cis-RA (data not shown). Similar induction (12 ± 1.0-fold) was observed when t-PA2.4-TK-CAT was co-expressed with both human RARβ and RXRα compared to RARβ alone. Combination of RA and 9cis-RA (both 10⁻⁶ M) did not cause additional induction, whereas 9cis-RA alone revealed a smaller increase. Similar induction was obtained with h-RARα instead of h-RARβ but not with h-RARγ (data not shown). When h-RXRα alone was co-expressed, maximal CAT induction required both RA and 9cis-RA (data not shown). Surprisingly, similar experiments with the t-PA9578-CAT construct, revealed no CAT induction at ratios of RARβ and RXRα expression vector to CAT reporter plasmid of 2 to 20. Apparently, expression of the RA nuclear receptors repressed the basal promoter activity of the t-PA reporter constructs (data not shown). However, such phenomenon was not observed when transient expression was performed in the EA.hy926 hybrid endothelial and the SK-N-SH neuroblastoma cell lines. The results obtained in these cell lines confirmed that the regulation of the t-PA promoter by RA is mediated by the t-PA/DR5 motif located in the t-PA2.4 enhancer (data not shown).

Figure 3A provides a schematic overview of the t-PA genomic sequence spanning -7,145 to -9,578 bp The indicated putative RAR/RXR binding sites are described in Table 1. Figure 3B represents the results of transient co-expression of t-PA2.4-TK-CAT mutants with RARβ and RXRα. Deletion of the DR0 motif (in t-PA2.0-TK-CAT and t-PA1.6-TK-CAT), of the DR4 motif (in t-PA1.4TK-CAT) and of the DR2 motif (in t-PA0.4-TK-CAT and t-PA2.4Δ0.5-TK-CAT) did not affect the induction by RA (9.3 ± 1.0-fold to 6.8 ± 0.03-fold) as compared to the full length t-PA2.4-TK-CAT construct (8.0 ± 0.8 fold). The t-PA1.9-TK-CAT construct, lacking the ER8 and DR5 elements, showed no CAT induction (1.1 ± 0.1-fold, whereas re-introduction of these elements in t-PA2.4Δ0.1-TK-CAT, restored the induction (7.4 ± 0.2-fold). The t-PA0.2-TK-CAT construct, containing the DR5 element but not the ER8 element, showed reduced RA induction (3.7 ± 0.1-fold). Elimination of the DR5 element by site-specific mutagenesis (t-PA2.0/DR5MUT-TK-CAT; for the exact sequence cfr. Table 1) abolished RA induction. Transient co-expression of CAT reporter constructs containing two copies of putative recognition motifs (Table 1) in front of the TK promoter revealed a 27 ± 7-fold and a 5.1 ± 0.2-fold induction for the t-PA/DR5 (t-PA/DR5-TK-CAT) and the t-PA/DR2 element (t-PA/DR2-TK-CAT), respectively (data not shown), compared to a 96 ± 22-fold induction observed for the DR5 element identified in the murine RARβ2 promoter used as a positive control (RARβ/DR5-TK-CAT,25). The distal glucocorticoid response element of the MMTV-Long Terminal Repeat (MMTV/GREa-TK-CAT,24) and the t-PA/ER8 element (t-PA/ER8-TK-CAT) conferred no induction (data not shown).

In conclusion, the t-PA2.4 genomic fragment contains two elements, t-PA/DR2 and t-PA/DR5, that confer RA-inducibility to the TK-promoter, but only t-PA/DR5 appears to be functionally active in the t-PA gene.

### Identification of the nuclear receptors involved in the response of the t-PA 2.4 enhancer to DEX and RA

### 1. Binding of GR and AR to the t-PA/GRE elements

The electrophoretic mobility shift assay (EMSA) was performed using a fusion protein of protein A with the DNA binding domain of either the glucocorticoid (GR) or the androgen receptor (AR). Both GR and AR, when stimulation is performed with their respective ligand, are equipotent transactivators of the t-PA2.4 enhancer (data not shown). A radiolabeled DNA fragment which spanned the t-PA genomic sequence from -7353 to -7,325 bp (t-PA0.2A) and which contained the t-PA/GREa element, showed strong binding in the presence of increasing amounts of GR and AR for which competition was observed with an increasing molar excess of MMTV/GREa oligonucleotide (data not shown). Figure 4A shows that GR specifically bound to a radiolabeled t-PA/GREa oligonucleotide (compare lane j to l with lane i), although with a lower affinity as for the consensus GRE from MMTV/LTR (MMTV/GREa, lane a to d). A t-PA/GREf oligonucleotide showed strong binding with GR (compare lane n to p with lane m) whereas t-PA/GREg showed a comparable affinity for GR as t-PA/GREa (compare lane q to t with lane i to l). An oligonucleotide combining both t-PA/GREf and t-PA/GREg with a spacing of 3 bp (as in their natural environment), showed strong binding (compare lane v to x with lane u) with the appearance of a slower migrating band which probably represents a GR-tetramer/probe complex (lane x). No binding was observed for a radiolabeled aspecific oligonucleotide (RARβ/DR5, lane e to h).

In conclusion, several GRE's were identified in the t-PA2.4 upstream genomic fragment, t-PA/GREa, GREf and GREg, which show specific affinity for GR and AR homodimers in vitro. However, the t-PA/GREf and t-PA/GREg element seemed not to be involved in the induction by DEX, whereas a role for t-PA/GREa remains to be confirmed by site-directed mutagenesis. The involvement of other putative sites present in this fragment has not been excluded.

### 2. Binding of RA receptors to the t-PA/DR5 RARE element

EMSA was performed using c-myc-epitope tagged RXRα (myc-RXRα) and flu-epitope tagged RARα (flu-RARα), the latter being equipotent to RARβ in transient expression assays (data not shown). A radiolabeled DNA fragment spanning t-PA genomic sequences from -7,145 to -7,324 bp (t-PA0.2B) and containing the t-PA/DR5 element, showed strong binding in the presence of both flu-RARα and myc-RXRα whereas with flu-RARα or myc-RXRα alone no binding was seen (data not shown). Displacement of the RAR-RXR complex was observed upon competition with a 10- and 100-fold molar excess of t-PA/DR5 oligonucleotide as well as with the positive control, the RARE sequence of the RARβ promoter (RARβ/DR5). Competition with an unrelated DNA oligonucleotide (MMTV/GREa) had no effect on complex formation. When EMSA was performed with a radiolabeled RARβ/DR5 oligonucleotide (positive control) similar results were obtained (data not shown)

In figure 4B, a radiolabeled t-PA/DR5 oligonucleotide did not interact with flu-RARα alone, weakly with myc-RXRα alone (lane i and j, respectively) and strongly with the mixture of both (lane k). Addition of specific monoclonal antibodies directed against the flu-epitope of RARα (anti-flu) or the myc-epitope of RXRα (anti-myc) caused a partial disruption of the retarded complex (lane l) and a clear supershift (lane m), respectively, suggesting that both RAR_{α} and RXR_{α} were present in the retarded complex. Similar results were obtained with the radiolabeled t-PA/DR2 and RARβ/DR5 oligonucleotides (cfr. Table 1), except that no interaction was observed with RXR_{α} alone (respectively lane o to u and lane a to g). A radio-labeled t-PA/ER8 oligonucleotide showed virtually no binding (data not shown) and experiments performed with a modified DR5 oligonucleotide, in which the first repeat of the DR5 motif was mutated but the ER2 repeats were left intact or a modified DR2 oligonucleotide in which only the ER1 was conserved (cfr. Table 1), revealed only weak binding of RARα/RXRα (data not shown).

In conclusion, two RARE elements were identified in the t-PA2.4 upstream genomic fragment (t-PA/DR5 and t-PA/DR2) with affinity for RAR-RXR heterodimers in vitro, although only the t-PA/DR5 element is functionally active in the t-PA gene.

### Effect of the distance from the transcription initiation site and of the intervening sequence on the induction of t-PA2.4 enhancer activity by DEX and RA

Stable expression of promoter deletion constructs containing the t-PA2.4 fragment (cfr. Figure 5A) in HT1080 cells, showed that the induction by DEX and RA alone (10⁻⁷ M) is not influenced by the distance or the intervening sequence while the synergistic effect of DEX together with RA (both 5X10⁻⁸ M) increased with decreasing distance between the enhancer and the t-PA proximal promoter elements (from 3.3 ± 0.06-fold for t-PA9,578-CAT to 7.3 ± 0.09-fold for t-PA632∇2.4-CAT, cfr. Figure 5B).

Experiments performed in HT1080 and in EA.hy926 and SK-N-SH cells confirmed that, respectively, the induction by DEX and RA was independent of the distance between the t-PA2.4 enhancer and the transcription start site (data not shown).

In aggregate, the response of the t-PA2.4 fragment to DEX and RA alone was independent of the intervening sequence and the distance to the proximal promoter elements. But, the synergistic effect of both hormones together increased with decreasing distance from the promoter.

### Synergy of steroid hormones with RA

Co-expression of the t-PA2.4-TK-CAT construct with different steroid hormone receptors in HT1080 cells revealed a similar induction for 10⁻⁶ M progesterone, 10⁻⁶ M d-aldosterone and 10⁻⁸ M synthetic androgen R1881 (6.5 ± 0,45-fold to 9.6 ± 0.85-fold) as compared to 10⁻⁶ M dexamethasone (8.8 ± 0,65-fold; the latter in the absence of co-expressed receptor), but no induction was observed with 10⁻⁶ M 17β-estradiol when the human estrogen was co-expressed (data not shown).

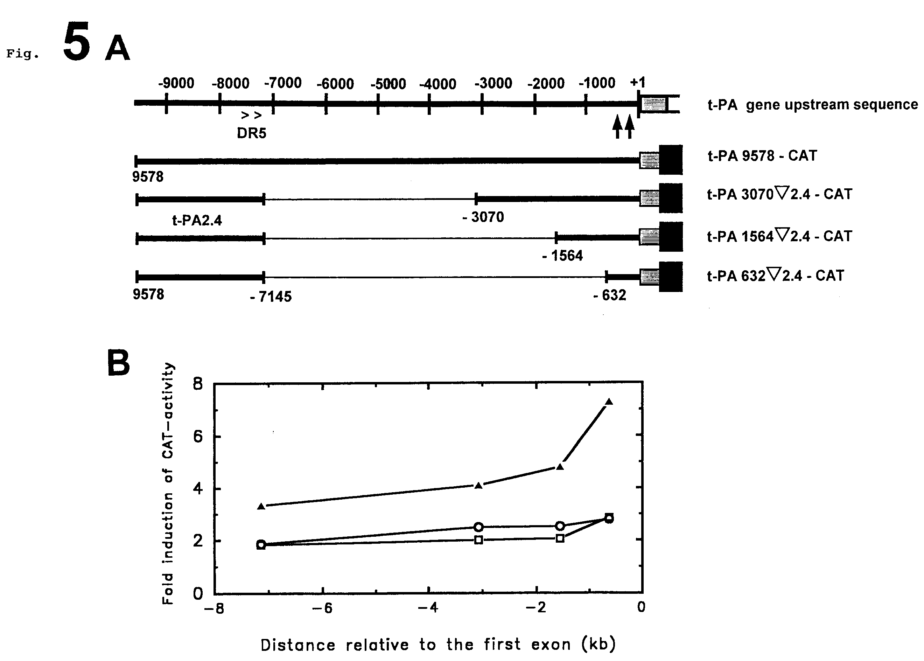

In order to determine whether the synergy observed between DEX and RA (see above) is unique or would also occur with other members of the steroid hormone family, co-expression was performed in HT1080 cells of the t-PA2.4-TK-CAT construct with the mineralocorticoid receptor together with the RARβ and RXRα receptors. Indeed, whereas treatment of d-aldosterone (ALD) and RA (both 10⁻⁷ M) induced CAT-activity respectively 3.0 ± 0.18 and 4.5 ± 0.11-fold, the combination of half of the ALD and RA concentration (both 5 x 10⁻⁸ M) resulted in a 8.4 ± 0.3-fold induction, which was significantly higher than would be expected for only an additive effect of these agents (cf. Fig. 6).

### Determination of the minimal functional enhancer size

As the cis-acting elements involved in the hormonal response of the t-PA2.4 enhancer are located at its 3' side, progressive 5' deletions of the t-PA2.4 enhancer linked to TK-CAT were evaluated by transient expression in SK-N-SK neuroblastoma cells. Deletion of the region spanning -9,578 to -8,559 bp (t-PA1.4-TK-CAT) and -9,578 to -8,006 bp (t-PA0.8-TK-CAT) did not affect the synergistic response to treatment with DEX and RA(both 5x10⁻⁷ M) as compared to the full size t-PA2.4-TK-CAT construct (respectively 6.8 ± 0.12-fold and 10 ± 0.45-fold as compared to 13± 0.54-fold, cfr. Figure 7). However, when the region spanning -9,578 to - 7,535 bp was deleted (t-PA0.4-TK-CAT), the induction by DEX was strongly reduced (1.3 ± 0.05-fold compared to 3.9 ± 0.14-fold for the wild-type t-PA2.4-TK-CAT construct) whereas the induction by RA was reduced by half (3.3 ± 0.09-fold compared to 6.2 ± 0.13). Moreover, the synergistic response to a combined DEX-RA treatment was eliminated (3.0 ± 0.13-fold compared to 13 ± 0.05-fold for the wild-type construct). In conclusion, these data show that the enhancer can be reduced to a minimal size of 863 bp spanning the nucleotides from -7,145 to -8,007 bp.

### Synergistic interaction between the t-PA2.4 enhancer and the t-PA proximal promoter

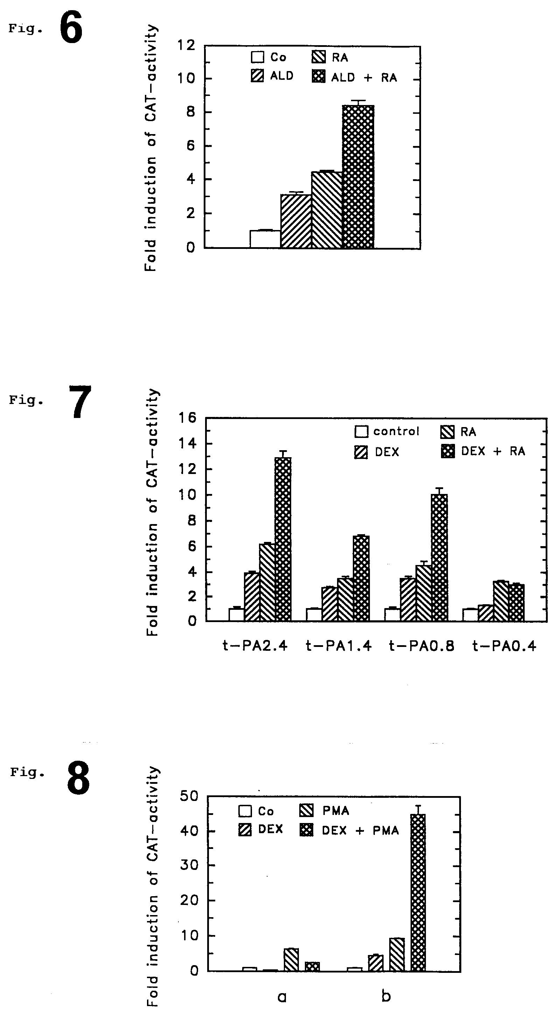

Stimulation of HeLa cells, transiently expressing the t-PA632-CAT and the t-PA632∇2.4-CAT reporter construct, with 160 nM phorbol-ester-myristate (PMA) resulted in a strong induction of CAT-activity (6.4 ± 0.13-fold and 17 ± 0.14-fold, respectively; see Figure 8A). DEX (10⁻⁷ M) induced the t-PA632∇2.4-CAT construct 4.5 ± 0.17-fold but had no effect on the t-PA632-CAT construct. Treatment with both DEX and PMA at 50% of their concentrations (5 x 10⁻⁸ M and 80 nM, respectively) resulted in a strong synergistic induction of transiently expressed t-PA632∇2.4-CAT (45 ± 3-fold) which was significantly higher than would be anticipated from only an additive effect of DEX and PMA. The induction of the t-PA632-CAT construct dropped by about two-fold compared to stimulation with PMA alone (2.5 ± 0.02-fold).

In conclusion, when linked to its homologous promoter (+ 197 to -632 bp), the t-PA2.4 enhancer provided a synergistic induction upon treatment with DEX and PMA in HeLa cells.

### LEGENDS TO THE FIGURES

- Fig. 1:: DEX- and RA-mediated transactivation of t-PA-CAT constructs following stable or transient expression in HT1080 human fibrosarcoma cells. The data represent mean ± SEM.
- Fig. 1A:: Schematic representation of the genomic sequences upstream from the transcription initiation site of the human t-PA gene and the t-PA-CAT-reporter constructs obtained thereof.
Numbering is according to Henderson and Sleigh (11). CRE/AP1 and AP2 binding sites involved in basal promoter activity (26) are indicated by an arrow and BamHI restriction sites are indicated by a filled triangle. The CAT reporter gene (CAT) (□), the TK-promoter (TK) (□) and the first exon (□) of the human t-PA gene are indicated.
- Fig. 1B:: Induction of CAT activity by DEX and RA (both 10⁻⁶M) by stably expressed human t-PA promoter constructs (a to f in the figure). Cells were harvested 24 hours after hormonal stimulation.
- Fig. 1C:: Synergistic induction of CAT activity by DEX and RA in HT1080 cells stably expressing the TK-CAT (d) and the t-PA2.4-TK-CAT construct (e). Cells were stimulated for 24 hours as indicated.
- Fig. 2:: Localization of the DEX responsive region in the human t-PA promoter by mutagenesis of the t-PA2.4 genomic fragment.
- Fig. 2A:: Schematic representation of the genomic sequence from -7145 to -9578 bp upstream from the human t-PA gene. Motifs resembling putative GRE's are represented (GREa, f, g).
- Fig. 2B:: Induction of CAT activity by DEX (10⁻⁶ M, hatched bars) versus control (open bars) of the indicated mutant constructs derived from t-PA2.4-TK-CAT. Hela cells, transiently co-expressing the reporter constructs with GR, were treated for 36 hours. The data represent mean ± SEM.
- Fig. 3:: Localization of the RA response element in the human t-PA promoter by mutagenesis of the t-PA2.4 genomic fragment. The data represent mean ± SEM of cells treated with RA (10⁻⁶ M, hatched bars) versus control (open bars).
- Fig. 3A:: Schematic representation of the genomic sequence from -7145 to -9578 bp upstream from the human t-PA gene. Motifs resembling repeats of the 
A   G   G
  G   T   A
G   T   C
 half site are represented: direct (DR) and everted repeats (ER), with the number of the intervening nucleotides, are indicated.
- Fig. 3B:: Induction of CAT activity by RA (10⁻⁶ M, hatched bars) of the indicated mutant constructs derived from t-PA2.4-TK-CAT. HT1080 cells, transiently co-expressing reporter constructs with h-RARβ and h-RXRα, were treated with RA for 36 h. The mutations in the DR5 element of t-PA2.0/DR5MUT-TK-CAT are marked with vertical bars (for corresponding sequence cfr. Table 1).
- Fig. 4:: Characterization of the GRE and RARE elements by the electrophoretic mobility shift assay using ³²P end labeled oligonucleotides.
- Fig. 4A:: Characterization of the t-PA/GRE using ³²P end labeled oligonucleotides representing MMTV/GREa, RARβ/DR5 and the t-PA/GRE elements identified at -7,501, -7,960 and -7,942 upstream from the human t-PA gene (respectively t-PA/GREa, t-PA/GREf and t-PA/GREg). Reactions were performed with fusion protein of protein A and the DNA binding domain of the glucocorticoid receptor (GR).
- Fig. 4B:: Characterization of the t-PA RARE using ³²P end labeled oligonucleotides representing the central RARβ/DR5, and the t-PA/DR5 and t-PA/DR2 elements identified at, respectively, -7,319 and -8,396 bp upstream from the human t-PA gene (for sequences, see Table 1). Reactions were performed with epitope tagged human RARα (flu-RARα) and RXRα (myc-RXRα) in the presence or the absence of an aspecific antibody (15C5) or specific antibodies (anti-flu and anti-myc). The difference in migration and relative intensity of the bands obtained for t-PA/DR2 were due to slightly different conditions applied during the electrophoresis of these reaction mixtures.
- Fig. 5:: Effect of the distance and the intervening sequence on the function of the t-PA2.4 enhancer.
- Fig. 5A:: Schematic representation of reporter constructs containing the t-PA2.4 fragment linked to various deletion mutants of the t-PA7144-CAT construct. The data represent mean ± SEM.
- Fig. 5B:: Response of t-PA-CAT constructs with increasing deletions between the t-PA2.4 enhancer and the promoter, stably expressed in HT1080 cells, to treatment with DEX and RA as indicated. Cells were treated for 24 hours with 10⁻⁷ M DEX (open squares), 10⁻⁷ M RA (open circles) or 5 x 10⁻⁸ M of both DEX and RA (filled triangle).
- Fig. 6:: Synergistic response of t-PA2.4-TK-CAT, stably expressed in HT1080 cells, to treatment with ALD and RA as indicated. Cells transiently co-expressed the human mineralocorticoid receptor and were stimulated for 24 hours with RA (10⁻⁶ M, □), ALD (10⁻⁶ M, □) or a combination of half of the RA and steroid hormone concentration (□), as compared to control (□). The data represent mean ± SEM.
- Fig. 7:: Delineation of the synergistic response of the t-PA2.4 enhancer by transient expression in SK-N-SH neuroblastoma cells. Cells were stimulated for 24 hours with DEX (10⁻⁶ M, □), RA (10⁻⁶ M, □) or a combination of DEX and RA (both 5 x 10⁻⁷ M, □) as compared to control (□). The data represent mean ± SEM.
- Fig. 8:: Effect of phorbol-ester-myristate (PMA) on the synergistic interaction between the t-PA2.4 enhancer and the proximal t-PA promoter elements. Response of t-PA632∇2.4-CAT (b) as compared to t-PA632-CAT (a), transiently expressed in HeLa cells, to treatment with DEX (10⁻⁷ M, □) or PMA (160 nM, □) or the combination of both DEX and PMA (5 x 10⁻⁸ M and 80 nM, respectively, □) as compared to control (□). Cells transiently co-expressed GR and were stimulated for 24 hours. The data represent mean ± SEM.

### REFERENCES

1. **Bayertz, K., R. Paslack, and K.W. Schmidt.** 1994 Summary of "Gene transfer into somatic cells. State of the technology, medical risks, social and ethical problems: A report." Human Gene Therapy, **5**: 465-468.
2. **Pabo, C.O. and R.T. Sauer.**1992 Transcription factors: structural families and principles of DNA recognition. Annu. Rev. Biochem. **61**, 1053-1095.
3. **Zawel, L. and D. Reinberg.** 1993 Initiation of transcription by RNA polymerase II: a multi step process. Progr. Nucleic Acids Res. Mol. Biol., **44**, 67-108.
4. **Evans, R.M.** 1988. The steroid and thyroid hormone receptor superfamily. Science **240**: 889-895.
5. **Beato, M.** 1989. Gene regulation by steroid hormones. Cell **56**: 335-344.
6. **Glass, C.K.** 1994. Differential recognition of target genes by nuclear receptor monomers, dimers and heterodimers. Endocrine Reviews **15**: 391-407.
7. **Holvoet, P., H. Cleemput, and D. Collen.** 1985. Assay of human tissue-type plasminogen activator (t-PA) with an enzyme-linked immunosorbent assay (ELISA) based on three murine monoclonal antibodies to t-PA. Thromb. Haemostas. **54**: 684-687
8. **Declerck, P.J., L. Van Keer, M. Verstreken, and D. Collen.** 1992. An enzyme linked immunosorbent assay for urokinase-type plasminogen activator (u-PA) and mutants and chimeras containing the serine protease domain of u-PA. Thromb. Haemostas. **67**: 95-100
9. **Declerck, P.J., M.C. Alessi, M. Verstreken, E.K.O. Kruithof, I. Juhan**-**Vague, and D. Collen.** Measurement of plasminogen activator inhibitor-1 in biologic fluids with a murine monoclonal antibody-based enzyme-linked immunosorbent assay. 1988. Blood **71**: 220-225
10**. Bulens, F., E.A. Thompson, J.M. Stassen, H. Moreau, P.J. Declerck, L. Nelles, and D. Collen.** 1992. Induction of t-PA synthesis with intravenous bolus injection of vitamin A palmitate in vitamin A deficient rats. Fibrinolysis **6**: 243-249.
11. **Henderson, B.R., and M.J. Sleigh**. 1992. TATA box-independent transcription of the human tissue plasminogen activator gene initiates within a sequence conserved in related genes. FEBS Lett **309:** 130-134.
12. **Luckow, B., and G. Schütz.** 1987. CAT constructions with multiple unique restriction sites for the functional analysis of eukaryotic promoters and regulatory elements. Nucleic Acids Res **15**: 5490.
13. **Higuchi, R.** 1990. Recombinant PCR, p. 177-183. In M.A. Innis, D.M. Gelfand, J.J. Sninsky and T.J. White (eds.), PCR protocols: a guide to methods and applications, Academic Press, San Diego, USA.
14. **Graham, F.L., and A.J. van der Eb.** 1973. Transformation of rat cells by DNA of human adenovirus 5. Virology **54**: 536-539.
15. **Bulens, F., A.-M. Vandamme, H. Bernar, L., Nelles, H.R. Lijnen, and D. Collen.** 1991. Construction and characterization of a functional chimeric murine-human antibody directed against human fibrin fragment D-dimer. Eur. J. Biochem. **195**: 235-242.
16**. Schwachtgen, J.-L., V. Ferreira, D. Meyer, and D. Kerbiriou-Nabias.** 1994. Optimization of the transfection of human endothelial cells by electroporation (Biotechniques, in press).
17. **van den Hoff, M.J.B., A.F.M. Moorman, and W.H. Lamers.** 1992. Electroporation in 'intracellular' buffer increases cell survival. Nucleic Acids Res. **20:** 2902
18. **Neuman, J.R., C.A. Morency, and K.O. Russian.** 1987. A novel rapid assay for chloramphenicol acetyltransferase gene expression. Biotechniques **5**: 444-447.
19**. Fried, M., and D.M. Crothers.** 1981. Equilibria and kinetics of lac repressor-operator interaction by polyacrylamide gel electrophoresis. Nucleic Acids Res. **9**: 6505-6525
20. **Baes, M., T. Gulick, H.-S. Choi, M.G. Martinoli, D. Simha, and D.D. Moore.** 1994. A new orphan member of the nuclear hormone receptor superfamily that interacts with a subset of retinoic acid response elements. Mol. Cell Biol. **14**: 1544-1551.
21**. Holvoet, P., J.M. Stassen, Y. Hashimoto, D. Spriggs, D. Devos, and D. Collen.** 1989. Binding properties of monoclonal antibodies against human fragment D-dimer of cross-linked fibrin to human plasma clots in an in vivo model in rabbits. Thromb. Haemostas. **61:** 307-313.
22. **Giguère, V., S.M. Hollenberg, M.G. Rosenfeld, and R.M. Evans.** 1986. Functional domains of the human glucucorticoid receptor. Cell **46**: 645-652..
23. **Brinkmann, A.O., P.W. Forbes, H.C.J. van Roojj, G.G.J.M. Kuiper, C. Ris, P. Klaassen, J.A.G.M. van der Korput, M.M. Voorhorst, J.H. van Laar, E. Mulder, and J. Trapman.** 1989. The human androgen receptor: domain structure, genomic organization and regulation of expression. J. Steroid. Biochem. **34**: 307-310.
24. **Kühnel, B., E. Buetti, and H. Diggelmann.** 1986. Functional analysis of the glucocorticoid regulatory elements present in the mouse mammary tumor virus long terminal repeat.J. Mol. Biol. **190**: 367-378.
25. **Hoffmann, B., J.M. Lehmann, X.K. Zhang, T. Hermann, M. Husmann, G. Graupner, and M. Pfahl.** 1990. A retinoic acid receptor-specific element controls the retinoic acid receptor-β promoter. Mol. Endocrinol. **4**: 1727-1736.
26. **Medcalf, R.L., M. Rüegg, and W.-D. Schleuning.** 1990. A DNA motif related to the cAMP-responsive element and an exon-located activator protein-2 binding site in the human tissue-type plasminogen activator gene promoter cooperate in basal expression and convey activation by phorbol ester and cAMP. J. Biol. Chem. **265**: 14618-14626.

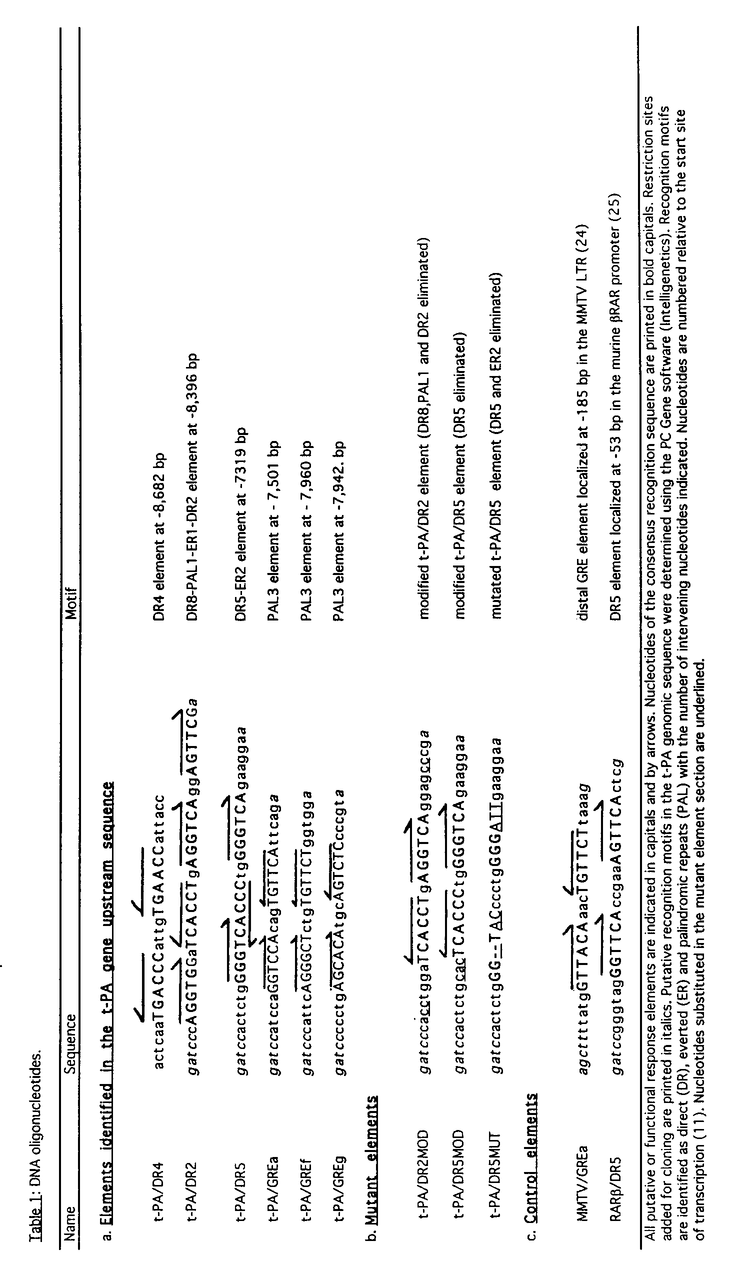

**Table 2**

| Influence of DEX (10⁻⁶ M) and RA (10⁻⁶ M) on t-PA, u-PA and PAl-1-related antigen secretion in the conditioned medium of HT1080 fibrosarcoma cells. | | | |
|---|---|---|---|
| Stimulation | t-PA | u-PA | PAl-1 |
| Control | 1.0 ± 0.05 | 1.0 ± 0.12 | 1.0 ± 0.1 |
| DEX | 2.9 ± 0.1 | 0.64 ± 0.02 | 2.4 ± 0.1 |
| RA | 11 ± 0.4 | 2.1 ± 0.17 | 1.3 ± 0.08 |
| DEX + RA | 35 ± 2.0 | 0.86 ± 0.05 | 3.0 ± 0.13 |
| Data were obtained by an antigen specific ELISA (see Materials and Methods) and were expressed as fold control (mean ± SEM). | | | |

## Claims

1. Enhancer element suitable for inducing or enhancing the transcription of cis-located promoter/gene constructs in response to steroids and/or retinoids, comprising at least the region spanning nucleotides -8,007 to -7,144 upstream of the transcription initiation site of human tissue-type plasminogen activator, or modified versions thereof not destroying the biological activity of the element.

2. Enhancer element as claimed in claim 1, wherein the element comprises the region spanning nucleotides -9,578 to 7,144.

3. Enhancer element suitable for enhancing the transcription of cis-located promoter/gene constructs in response to steroids and/or retinoids, comprising a steroid responsive-fragment located in the region spanning nucleotides -7,325 to -7,535 and/or a retinoic acid responsive-site located in the region spanning nucleotides - 7,145 to -7,324.

4. Enhancer element as claimed in any one of the claims 1-3 comprising at least one biologically active part of the DNA sequence shown in Fig. 9, or modified versions thereof not destroying the biological activity of the element.

5. Vector for the transfer of a gene or gene fragment into mammalian host cells, such that the gene or gene fragment may be expressed by the host cell, the vector comprising an enhancer element as claimed in any one of the claims 1 to 4, operably linked to its homologous or any heterologous promoter and the gene or gene fragment.

6. Vector as claimed in claim 5 for use in the in vivo modulation of the expression of the gene or gene fragment in mammalian host cells, by natural or synthetic steroids, retinoids or combinations thereof.

7. Vector as claimed in claim 5 for use in the in vivo modulation of the expression of the gene or gene fragment in mammalian host cells, by one or more agents acting on the enhancer element, such as activators of protein kinase C.

8. Use of a vector as claimed in claim 5 for the in vivo modulation of the expression of the gene or gene fragment in mammalian host cells, by natural or synthetic steroids, retinoids or combinations thereof.

9. Use of the vector as claimed in claim 5 for the in vivo modulation of the expression of the gene or gene fragment in mammalian host cells, by one or more agents acting on the enhancer element, such as activators of protein kinase C.

10. Genetic switch, comprising at least part of the enhancer element as claimed in any one of the claims 1-4 and a promoter having substantially no expression level of its own, wherein the enhancer element and the promoter are located on the same vector or on separate vectors that are used simultaneously.
